Europäisches Patentamt

European Patent Office

Office européen des brevets

(11) Publication number: **0 171 881**
**A1**

## (12) EUROPEAN PATENT APPLICATION

(21) Application number: **85303924.6**

(22) Date of filing: **04.06.85**

(51) Int. Cl.⁴: **A 61 N 1/36**

(30) Priority: **05.06.84 US 617444**

(43) Date of publication of application:
**19.02.86 Bulletin 86/8**

(84) Designated Contracting States:
**DE FR GB IT SE**

(71) Applicant: **CODMAN & SHURTLEFF INC.**
**Pacella Park Drive**
**Randolph Massachusetts 02368(US)**

(72) Inventor: **Mickiewicz, Stanley P.**
**147 Wheeler Circle Apt. 224**
**Stoughton, MA 02062(US)**

(72) Inventor: **Coombes, Alan**
**19 Volunteer Road**
**Hingham, MA 02043(US)**

(74) Representative: **Jones, Alan John et al,**
**CARPMAELS & RANSFORD 43 Bloomsbury Square**
**London, WC1A 2RA(GB)**

(54) Treatment pulse sequences for a transcutaneous nerve stimulation device.

(57) A transcutaneous nerve stimulation device is provided for producing pulse sequences in a modulation mode and a strength-duration mode. In the modulation mode, the amplitude and pulse width are modulated during successive sequences of pulses of different pulse rates. In the strength-duration mode, the operator inputs the amplitudes of two pulses of preselected pulse width into the stimulation device. The device then automatically calculates a strength-duration curve which is fitted to the parameters of the two pulses. By varying the displayed pulse width parameter, the device will produce a treatment pulse sequence of the displayed pulse width and corresponding pulse amplitude of the strength-duration curve.

./...

EP 0 171 881 A1

FIG-1

# TREATMENT PULSE SEQUENCES FOR A TRANSCUTANEOUS NERVE STIMULATION DEVICE

This invention relates to transcutaneous nerve stimulation devices and, in particular, to treatment pulse sequences for such devices.

Transcutaneous nerve stimulation devices apply stimulating electrical pulses to electrodes which contact the skin of a patient to relieve pain through electrical nerve stimulation. A patient will generally be given a device by a physician, together with instructions on how to use the device safely and effectively. Thereafter, the patient uses the device to perform the necessary treatment. Accordingly, it is desirable for the device to be simple and easy for the patient to use.

It has been found that different patients receive treatment benefit from the application of different types of pulse sequences to differing areas of the body. Some patients may benefit from a continuous sequence of pulses of constant intensity and duration. Others may benefit from periodic bursts of short pulse sequences, for instance. Hence, a stimulation device should be capable of providing a range of different types of pulse sequences so that the physician can prescribe the treatment pulse sequence most beneficial to any particular patient.

Moreover, the physician should be able to tailor the parameters of any type of pulse sequence when searching for the exact sequence most beneficial for any patient. That is, the pulse intensity, duration and rate should all be controllable by the physician as he or she searches for the most desirable pulse sequence.

However, it may not be possible for the physician to pinpoint the exact pulse sequence which will provide continuous benefit over an extended treatment period. In such cases it is desirable to prescribe a pulse sequence which exhibits a range of differing stimulation characteristics, with the expectation that one or more of the characteristics will provide the desired benefit. Such a pulse sequence is called a modulation pulse sequence. It is desirable for such a sequence to exhibit variation in all of the controllable sequence parameters: intensity, duration and pulse rate.

In accordance with the principles of the present invention, a modulation mode of operation is provided in a stimulation device during which the intensity, or pulse amplitude, the pulse duration, or width, and the pulse rate are controllably varied during treatment. In a preferred embodiment, to begin treatment, the patient controls the device by setting the parameters of a single starting pulse, including pulse amplitude, width and rate. From this starting pulse, the stimulation device automatically calculates the changes in the pulse sequence parameters which occur continuously as treatment progresses.

In the preferred embodiment, the device produces a first pulse sequence at a first pulse rate. The pulses of the first sequence exhibit different pulse amplitudes and widths. The first pulse sequence is followed by a second sequence at a second pulse rate, which also exhibits pulses of varying amplitudes and widths.

The second sequence is succeeded by a third pulse sequence of a third pulse rate, again with differing pulse widths and amplitudes. Thereafter the process is reversed, with the device producing the second and first sequences. This cycle of sequences is then repeated continuously during treatment.

It has been established that, for any sequence of pulses of constant amplitude and width, there is a family of pulses with different amplitudes and widths which will provide the same treatment stimulus. The relation between these different amplitudes and widths is described by a strength-duration characteristic curve. The strength-duration curve is a logarithmic curve in accordance with a known mathemetical expression. It is desirable for a physician, upon finding a beneficial pulse sequence, to be able to experimentally try other pulse sequences in the strength-duration family so as to prescribe the most beneficial pulse sequence.

In accordance with a further aspect of the present invention, a strength-duration mode of operation is provided in a stimulation device for clinical utility by the physician. In this mode of operation, the physician enters the amplitudes and widths of two pulses into the device. The device will then calculate the strength-duration curve which is characteristic of the two pulses. By changing the value of a pulse parameter the physician is able to produce sequences of different pulses of the strength-duration curve.

In the drawings:

FIGURE 1 is a schematic illustration of a stimulation device constructed in accordance with the principles of the present invention;

FIGURES 2, 3, and 4 are more detailed schematics of the illustration of FIGURE 1;

FIGURE 5 is a block diagram of the controller of FIGURE 1;

FIGURES 6A, 6B, 6C and 7 are flowcharts of the programming of the controller of FIGURE 5;

FIGURE 8 illustrates the pulse parameter relationship of modulation mode pulses;

FIGURE 9 illustrates a modulation mode pulse sequence;

FIGURE 10 illustrates a strength-duration curve; and

FIGURE 11 illustrates the operation of the stimulation device of the present invention during the strength-duration mode.

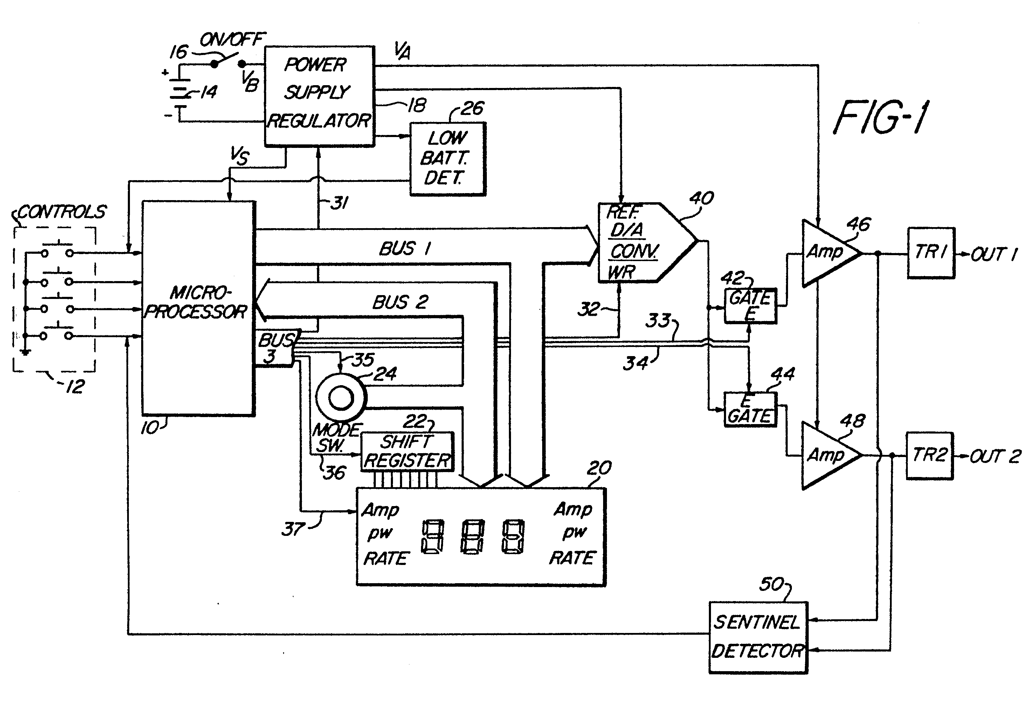

Referring to FIGURE 1, a schematic illustration of a digitally-controlled stimulation device constructed in accordance with the principles of the present invention is shown. A microprocessor 10 receives operator input control signals from pushbutton switches shown at 12. The microprocessor also receives inputs from a low battery detector 26 and a sentinel signal detector 50. Signals from either of these latter two sources will precipitate a termination of output pulse production.

The device is powered by a battery 14, which is connected to a power supply regulator 18 by an on/off switch 16. The regulator 18 provides regulated supply voltage $V_S$ for the microprocessor 10, output stage voltage $V_A$, and a reference voltage for a digital-to-analog (D/A) converter 40. The regulator is also coupled to the low battery detector 26 for voltage sampling by the detector.

The microprocessor 10 has two ports and a data bus, shown as bus 1, bus 2, and bus 3. Bus 1 provides digital words representative of output signal levels to the D/A converter 40, and also control signals for segments of the right-most digit of a display 20. Bus 2 provides control signals for segments of the center digit of the display 20, and also samples the setting of a mode switch 24. Bus 3 has a plurality of data lines for dedicated control functions. Line 31 controls the timing of the application of the reference voltage to the D/A converter 40. Line 32 controls the clocking of an output voltage level word into the buffer of the D/A converter. Lines 33 and 34 control the closings of gates 42 and 44, which couple the voltage from the D/A converter to the outputs of the device. Line 35 applies a signal to the mode switch 24 during the time that the state of the mode switch is sampled. Line 36 applies input data to a shift register 22, and line 37 turns on the AMP indicator on the left side of the display 20 when the amplitude of pulses at the first output is being displayed numerically.

The output of the D/A converter is coupled by way of the two CMOS gates 42 and 44 to inputs of output signal amplifiers 46 and 48. The outputs of the amplifier 46 and 48 are coupled to respective output transformers TR1 and TR2, and to inputs of the sentinel signal detector 50.

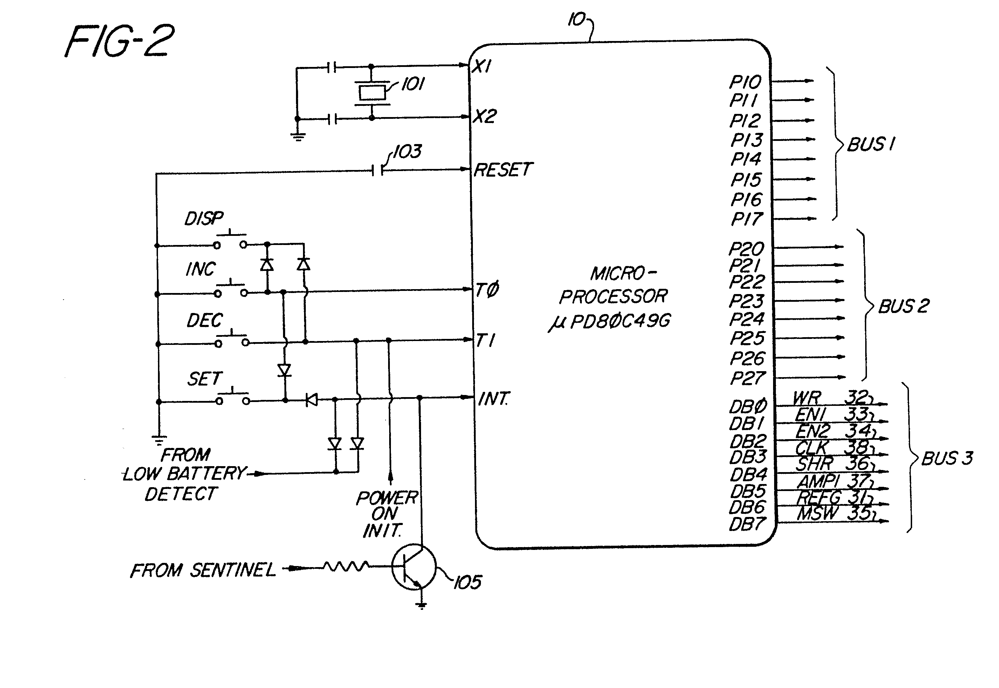

A portion of the schematic diagram of FIGURE 1 is shown in further detail in FIGURE 2. The microprocessor 10, which may be of the generic type designated 80C49, is shown with its inputs and outputs specifically designated. A 3 MHz crystal 101 is coupled across inputs X1 and X2 of the microprocessor 10. A capacitor 103 couples the reset input of the microprocessor to ground so that the microprocessor will receive a reset command immediately after the device is turned on and the supply voltage has stabilized. Four pushbutton controls, labelled DISP, INC, DEC and SET are coupled to inputs $T\emptyset$, Tl and the interrupt input of the microprocessor. A number of diodes are used to encode signals from the pushbutton switches. For instance, when the DISP pushbutton is closed to change the function being displayed on the display, the diodes from the DISP pushbutton apply low (ground level) signals to the $T\emptyset$ and Tl inputs. The microprocessor will then decode these two low lines as a DISP command. Also coupled to microprocessor inputs are a sentinel signal by way of transistor 105, and a low battery detection signal. A power on initializing signal is coupled to input Tl.

The individual lines of the three buses are also shown. Buses 1 and 2 are ports of eight lines each, and bus 3 comprises eight data lines for controlling the specific functions enumerated above. Line 38, which was not shown in FIGURE 1, is a clock line which is selectively activated to load data from line 36 into the shift register 22.

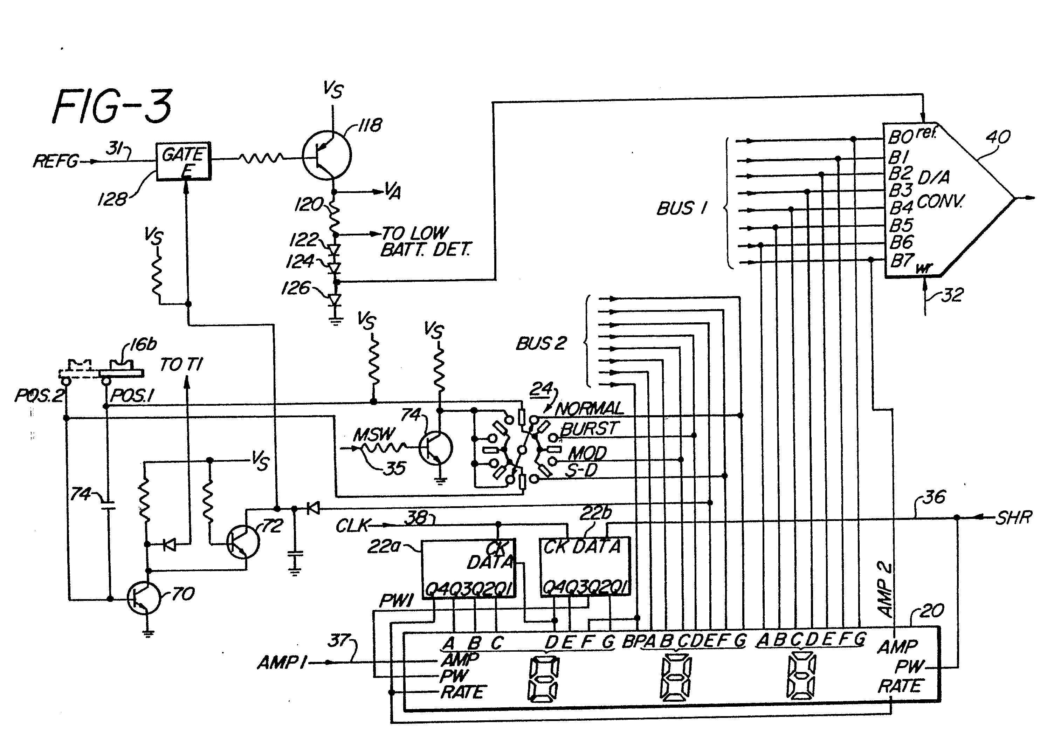

FIGURE 3 illustrates the display and bus arrangement of FIGURE 1 in further detail. Line 31 from the microprocessor is coupled through a CMOS gate 128 to the base of a transistor 118. A signal REFG on this line 31 turns on transistor 118 to apply a reference voltage to D/A converter 40 when needed during activation of the D/A converter. When the D/A converter is not supplying an output voltage, the reference voltage is not needed and is turned off by the REFG signal to conserve battery power. The reference voltage is otherwise turned on momentarily every half second to sample the battery voltage. The gate 128 will be interrupted momentarily after the on/off switch 16 is turned on while capacitor 74 charges. The time for the capacitor to charge allows the system to stabilize before transistor 70 is turned off, at which time the T1 input of the microprocessor is released. When T1 is released, the output pulses are at a low, safe level. Transistor 72 in turn will turn off, releasing the enabling line to gate 128. Transistor 72 also forces a mode change to the desired mode. The application of reference voltage to the A/D converter may then proceed, as the microprocessor is running with valid data. This hold-off sequence prevents the application of inaccurate, high-level output pulses to the patient after a momentary turning off of power, which could leave spurious data in the microprocessor registers until the registers have been validly restored.

When the mode switch 24 is to be interrogated to determine if a change in mode has occurred, a signal MSW on line 35 momentarily turns on a transistor 74. The arm of the mode switch 24 will then apply a low signal level to the output of the switch to which it is presently connected. This low signal level is detected on the corresponding one of the four lines of bus 2 to which the mode switch is connected. Bus 2 is acting as a data input port at that time. During the remainder of the time, bus 2 is providing segment drive signals for the middle digit of the display 20 and a backplane (BP) signal. Bus 1 is providing segment drive signals for the right-most digit of the display at all times except when the D/A converter 40 is being loaded with output signal level data.

Shift register 22, which is comprised of two four-bit, serially connected shift registers 22a and 22b, is loaded with data for the left-most display digit from line 36. The clock signal on line 38 shifts the data into the shift registers 22a, 22b. The display also displays the indicators AMP, PW, and RATE on either side to tell the operator which parameter is being controlled by the INCREASE and DECREASE pushbuttons. The left indicators correspond to output 1 and the right indicators to output 2. The AMP indicator for output 1 is controlled by the AMP1 signal on line 37, and the AMP indicator for output 2 is controlled by one line of bus 1. The PW (pulse width) indicator for output 1 is controlled by the Q2 location of shift register 22b, and the PW indicator for output 2 is time-shared on line 36. The RATE indicators are commonly controlled by the Q4 location of shift register 22a, as both outputs will exhibit the same pulse rate, or frequency.

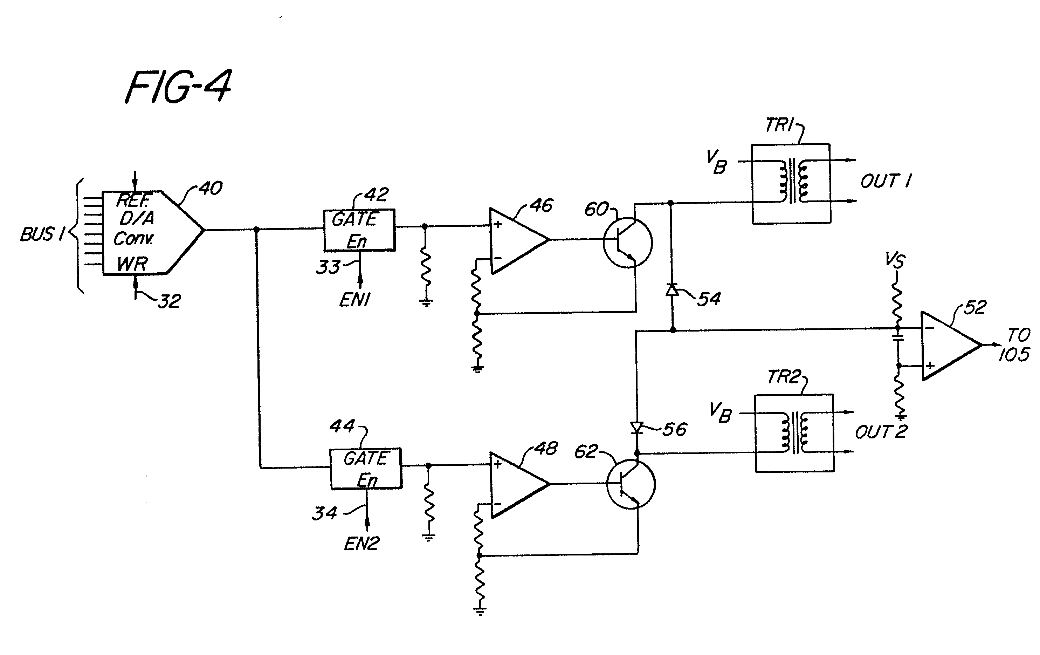

The output section of the stimulation device is shown in detail in FIGURE 4. When the D/A converter is producing the desired output level, signals EN1 and EN2 on lines 33 and 34 render CMOS gates 42 and 44 conductive for periods corresponding to the operator-entered pulse width settings. Output pulses are then applied to amplifiers 46 and 48. Output transistors 60 and 62 are turned on to apply output signals to output transformers TR1 and TR2. The output pulses are then transformer coupled to leads connected to electrodes (not shown) on the skin of the patient.

Before each transmission of output pulses, test pulses called sentinel pulses are transmitted to verify that the electrodes are properly connected to the patient. If the connections are properly made, the pulses will not exceed the trigger threshold of a comparator 52, and the output state of the comparator will not change. Improper electrode connections will cause detection of the sentinel pulses, which are coupled to the interrupt input of the microprocessor by transistor 105 (FIGURE 2), and the microprocessor will be informed of the improper connections. The microprocessor will query its interrupt line, and will recognize the problem. A sentinel flag will be set, and the system will halt the production of output pulses until the failure is remedied and the device is restarted. The sentinel system is more fully described in concurrently filed European patent application number _____, entitled "TRANSCUTANEOUS NERVE STIMULATION DEVICE WITH SENTINEL" (Reference STM 27, claiming priority from USSN 617 429).

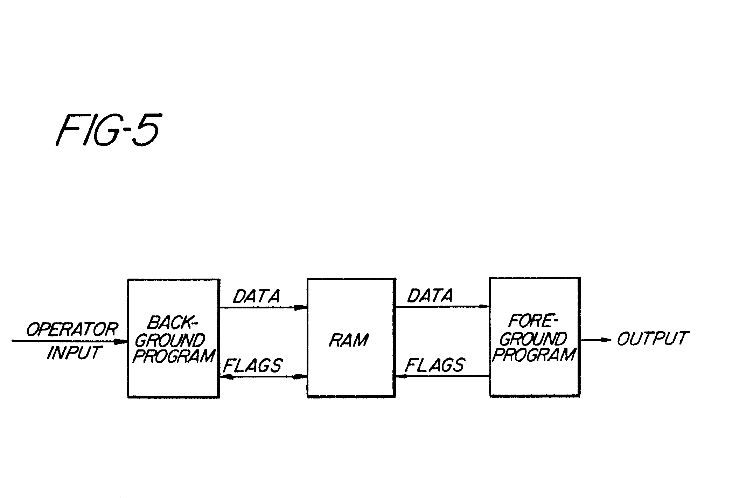

The software which controls the microprocessor, and hence operation of the device, is stored in a 2K read only memory (ROM). The software is partitioned into a foreground program and a background program. The two programs interact through an exchange of data words and flag bits stored in 128 bytes of random access memory (RAM). This software/hardware structure is shown in FIGURE 5. During most of the time, the background program loop is running. The background program continuously monitors the pushbutton inputs and the mode switch, looking for a change. When a change occurs, it is detected and any variables which require change are calculated and stored in the RAM. The variables are then available for use by the foreground program.

An internal timer which runs continuously interrupts the background program every 2.5 msec. The foregound program, which controls precise timing functions, then proceeds through its sequence, producing output signals at the appropriate times. Output signals are produced in accordance with the data values that the foreground program finds in RAM locations. When the foreground program produces output signals from new data values it returns status flags to the RAM to indicate to the background program that the new data has been utilized.

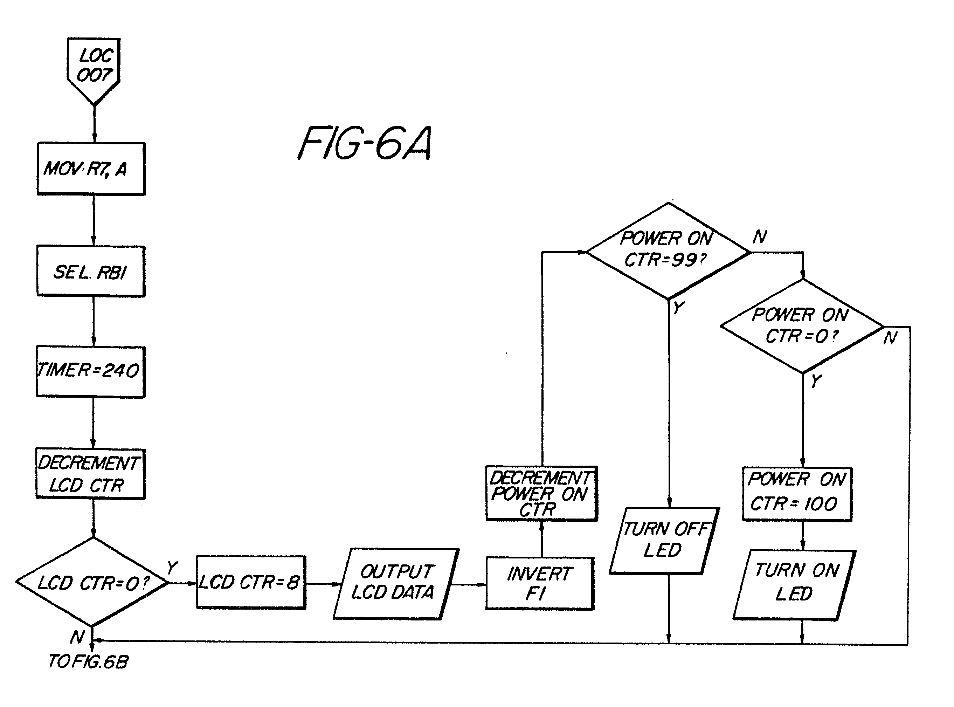

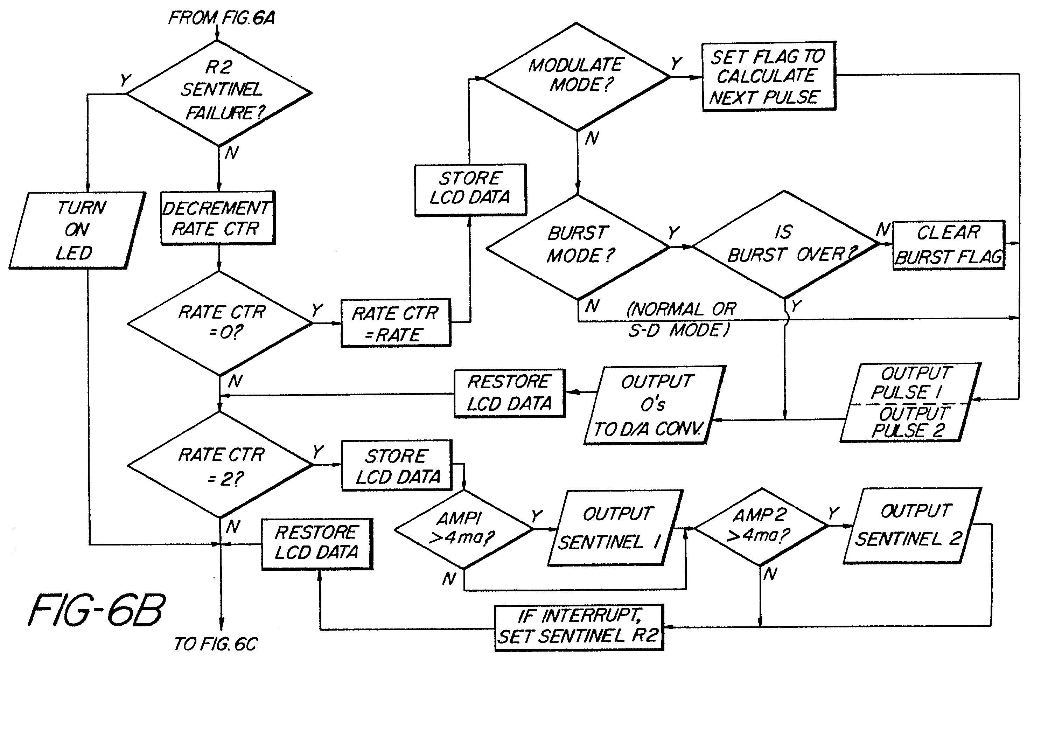

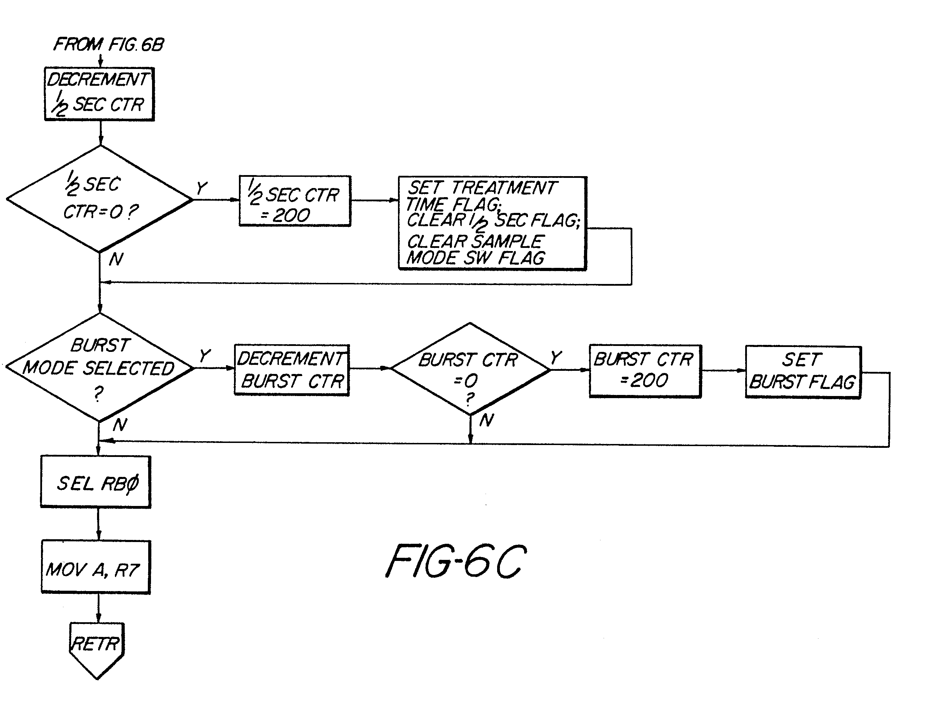

The foreground program is shown in flowchart form in
FIGURES 6A, 6B and 6C. When the internal timer has
reached zero after 2.5 msec, the background program is
interrupted and the microprocessor goes to location 007,
where the foreground program begins. The background program data in the accumulator is stored in register 7, and
register bank 1 is selected for foreground program control. Register bank 1 contains a number of counters which
are decremented by the foreground program to maintain the
appropriate timing of output functions. The internal
timer is initialized to a value of 240, corresponding to
2.5 msec, and the foreground processing begins.

The foreground program first decrements an LCD counter for
control of the display. The LCD display receives two
types of pulse trains: a common backplane pulse train and
individual segment pulse trains, each of a frequency of
25 Hz. When the two pulse trains are changing in phase
for any one display segment or indicator, there is no
potential difference between the pulse trains and the
segment or indicator is not displayed. When the two pulse
trains are in phase opposition, however, there is always a
voltage differential between them and the segment or
indicator will be displayed.

When the LCD counter has been decremented to zero, a half
cycle of the 25 Hz pulse rate has expired and the outputs
to the LCD display must be changed. The LCD counter is
initialized to a value of eight (8 times 2.5 msec =
20 msec = 1/2 cycle of 25 Hz), and LCD data resident in
the RAM is put on buses 1 and 2. LCD data is serially
loaded into the shift register 22 from data line 36. An
invert flag F1 is set to inform the background program
that it must calculate inverted LCD data values for the
next half cycle (opposite phase) of the LCD pulse trains.

A Power On counter is decremented. Every two seconds a "power on" LED (light emitting diode) is flashed for 20 msec to indicate to the operator that the device is operating properly. The counter is first checked for a value of 99. At this value, the LED is turned off. The counter is then checked for a value of zero, at which time the LED is to be turned on. At zero the counter is reinitialized to a value of 100, the LED is turned on, and the foreground program continues in FIGURE 6B.

A register R2 is then checked to see if a sentinel failure has been detected. If so, the LED is turned on continuously to alert the user of the problem. The LED can only be turned off by remedying the electrode problem and restarting the system.

The program next decrements the rate counter. The rate counter counts down from an initial value (RATE) which is a number calculated by the background program. The RATE number represents the interval between output pulses selected by the operator. If the rate counter has reached zero, output pulses are to be produced, and the counter is reinitialized and the LCD output data is stored. Since bus 1 will now be used for output data, the LCD data on that bus must be stored during this time, and restored on the bus after the output pulse sequence is finished.

The program first checks to see if the device is operating in the modulate mode. In this mode, each output pulse differs in pulse width and amplitude from the previous pulse. Hence, a flag is set to inform the background program that a pulse has been produced, and that new parameter values must be calculated for the following pulse. If the device is not in the modulate mode, the program checks to see if the device is in the burst mode. In the burst mode, a burst of seven pulses is produced every half second at a rate of 80 Hz. The amplitude of the burst pulses is determined by the operator. If the device is in the burst mode, the program checks to see if a pulse sequence is in progress. If the burst pulse sequence is over, no output is produced. If the sequence of seven pulses is not complete, a burst mode flag is cleared and pulse output commences.

When the program does not find the device to be operating in either the modulate or burst mode, the device must be operating in either the normal or the strength-duration mode. In either case, a repetitive sequence of treatment pulses is to be produced, and the program proceeds to produce output pulses.

The microprocessor controls pulse production at the first output, then pulse production at the second output. At first, a REFG signal on line 31 applies a reference voltage to the D/A converter. A digital word is put on bus 1, and is loaded into the D/A converter buffer by a WR signal on line 32. An EN1 signal on line 33 then renders gate 42 conductive for the desired duration, or pulse width, and the output signal is produced at the first output. The program then loads a new pulse amplitude value into the D/A converter, and the gate enabling sequence is performed on gate 44 to produce the selected pulse at the second output. After pulse output is complete, the REFG signal is dropped, and all zeroes are loaded into the D/A converter. The LCD data is restored on bus 1 for the display, and the foreground program continues. Pulse output comprises a major portion of the foreground program's time, since output pulses can range from 30 to 250 microseconds in duration.

The program next checks to see if the count of the rate counter is two. If so, sentinel pulses must now be

'M-28

produced, in advance of the output pulses five milliseconds later. Again, the LCD data on bus 1 is stored so that bus 1 can be used to transfer pulse amplitude values to the D/A converter. The program checks to see if the amplitude of the upcoming pulse for the first output will be greater than 4 milliamperes as referenced to an internal milliampere scale. If not, no sentinel is needed, since the output pulses themselves are too low to result in any patient hazard. But if the output pulse is to be greater than 4 ma., a sentinel pulse with a 5 ma. amplitude and a 30 µsec. duration is produced at the first output, using the same output sequence described above. This process is repeated to generate a sentinel pulse at the second output.

If an electrode has come loose from the patient as detected by the comparator 52, an interrupt will have occurred at the INT input. If the interrupt has occurred, a sentinel-detected failure flag is set in a register R2. During the following pass through the foreground program, this flag in register R2 will be detected, the LED will turn on, and the output pulse sequence will be bypassed.

Continuing on in FIGURE 6C, a 1/2 second counter is decremented. A number of functions occur at half-second intervals. If the 1/2 second counter is at zero, it is reinitialized and a treatment time flag is set. During treatment, the patient sees treatment time on the display in elapsed time up to 255 minutes. Treatment time is accumulated in half-second intervals by the background program in response to the setting of the treatment time flag.

A half-second flag is also cleared. To prevent a patient from producing needlessly high amplitude pulses by ramping the output pulse amplitude up too quickly, the pushbuttons that permit the pulse parameters to be changed are allowed to produce a step change only once each half-second. This half-second flag allows these time increments to be monitored by the background program. When a parameter pushbutton is held down, the parameter will change by one increment every half second.

A sample mode switch flag is also cleared. This flag tells the background program to interrogate the mode switch every half second. More frequent interrogation has not been found to be helpful or necessary, and in fact reduces the time during which other functions may be performed.

After the 1/2 second counter is queried, the mode is again checked to see if the burst mode has been selected. If it has, the burst counter is decremented and checked to see if it is at zero. If so, it is reinitialized and the burst flag is set so that a new sequence of seven burst pulses will be produced beginning with the next pass through the foreground program.

The foreground program is now completed, and the register bank ∅ for the background program is selected. The accumulator is restored to the contents it had at the beginning of the foreground sequence, and the system resumes with the background program at the point where it was before the foreground timer interrupt occurred.

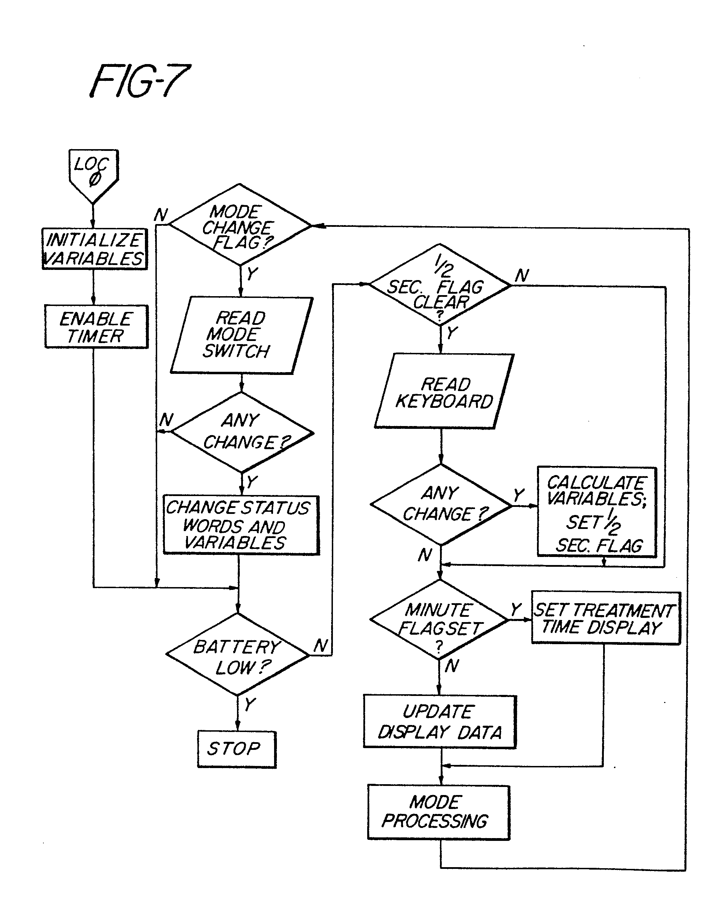

The background program is shown in the flowchart of FIGURE 7. When the stimulation device is turned on, the microprocessor is reset and the background program starts from location zero. Several variables are initialized; pulse amplitude is set to zero, pulse width is set at 50 μ sec., and the rate is set at 80 Hz. The sentinel is cleared and counters are initialized. The internal timer for the foreground program is enabled with its initial count of 240.

The background program then checks the battery voltage. If it is low, the program stops and the LED is lit. If the battery voltage is at a sufficient level, the program checks to see if the one-half second flag has been cleared by the foreground program. If the flag has been cleared, the background program reads the keyboard buttons DISP, INC, and DEC, and looks for any change. If the flag has not yet been cleared, a half second has not passed since the last button was pushed, and the keyboard is not read. If a new button entry is detected, the program decodes and notes it, and calculates the appropriate new variables for storage in the RAM. After this calculation, the one-half second flag is reset, to be cleared by the foreground program after one-half second has been counted down.

The background program then checks to see if a minute flag has been set. During treatment, the program records and displays treatment time in minutes. Treatment time is tracked in one-half second increments. When a full minute has been accumulated, the treatment time display is incremented to the next minute, up to a maximum of 255 minutes. During treatment this time is flashed periodically to inform the user that treatment is progressing normally. The off time during flashing extends the life of the display.

After the minute flag is checked and found not to be set, the data for the LCD display is calculated and loaded into the appropriate locations in the RAM for access by the foreground program. As mentioned above, the LCD display waveforms must be inverted every 20 msec, in synchronism

with the change of state of the LCD backplane pulse train of 25 Hz.

Thereafter, the background program does processing necessary according to the mode of operation. If treatment is proceeding in the normal or the strength-duration mode, the program proceeds to the mode interrogation sequence of the program. If the device is in the burst mode, the program checks to see if the burst sequence of seven pulses has been completed. If the device is in the modulation mode, the program calculates the parameters of the next pulse if the appropriate flag has been set by the foreground program. If the device is in the calibration portion of the strength-duration mode, the program calculates any needed pulse widths, amplitudes, and the logarithmic curve for pulse characteristics. After these calculations, the program proceeds to the mode interrogation sequence.

The background program next checks to see if the sample mode switch flag has been cleared by the foreground program. If it hasn't, the program drops down to the battery check step. If the flag has been cleared, the program reads the mode switch. If there has been a mode change, the program changes any status words for the mode processing sequence, including the flag, and initializes variables for parameter values. The background program then proceeds to the battery check step.

It should be remembered that the foreground program is continually interrupting the background program every time the internal timer counts an interval of 2.5 milliseconds.

In the modulation mode of operation, the patient can enter the parameters of starting pulses for each of the two outputs. Since this sequence is the same for both outputs, operation in the modulation mode will be described for only one output.

The patient begins by setting the mode switch 24 to the modulation mode and turning on the stimulation device. The AMP indicator for one output will be displayed and the patient will press the INC and DEC buttons until the desired starting pulse amplitude is displayed. This is the highest amplitude pulse produced during the modulation mode.

Next the patient presses the DISP button to display the PW indicator for the channel, and the width of the starting pulse is entered. The DISP button is pushed again to display the RATE indicator, and the starting pulse rate in pulses per second is entered. The stimulation device then begins to produce treatment pulses.

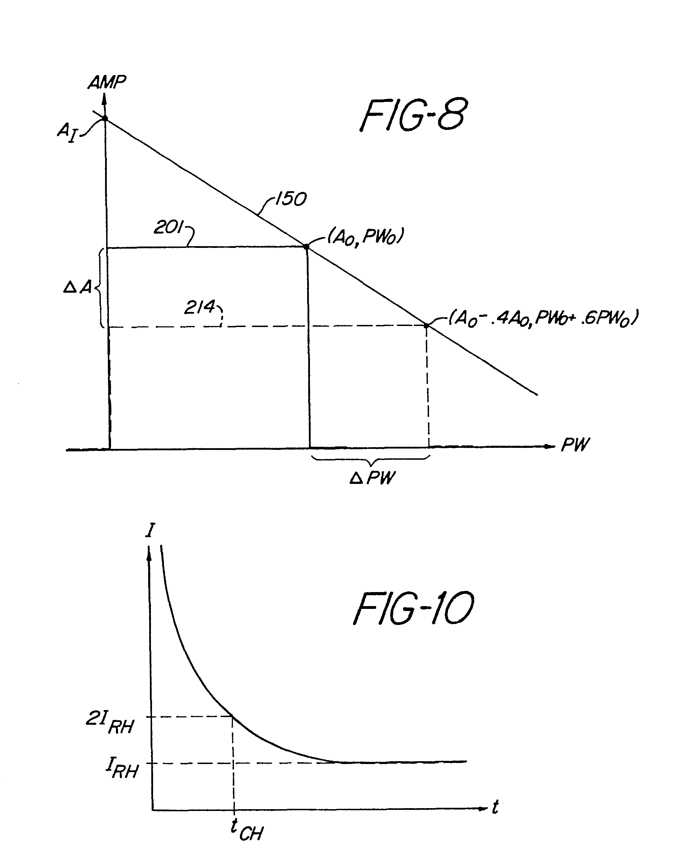

During treatment, the background program calculates parameters for the output pulses as they are needed based upon the starting pulse parameters. Referring to FIGURE 8, an example of a starting pulse is shown. This starting pulse 201 is depicted on an amplitude versus pulse width plot starting at the origin, and has an amplitude value of $A_0$ and a pulse width value of $PW_0$. During the modulation mode, fourteen pulses of successively decreasing amplitude and increasing pulse width are produced. The fourteenth pulse in this succession exhibits an amplitude decreased by 40% from the amplitude of the starting pulse, and a pulse width which is 60% greater than that of the starting pulse. This fourteenth pulse 214, then, has an amplitude of $A_0-.4A_0$, and a pulse width of $PW_0+.6 \, PW_0$.

The amplitude and pulse width points of pulses 201 and 214 are connected by a straight line 150 in FIGURE 8. The amplitude and pulse width parameters of the twelve intervening pulses are located at evenly spaced locations on line 150, between the two illustrated parameter points. Once the parameter points for pulses 201 and 214 have been determined, the difference between the respective amplitude values, $\Delta A$, and the difference between the respect pulse width values, $\Delta PW$, can be found and divided by fourteen to find the parameters of the twelve intervening pulses. Alternatively, the equation for a straight line, $y = mx + b$, can be solved using the two known parameter points. The slope $m$ of the line is always equal to $-2/3$ by the -40% to +60% relation of the amplitude and pulse width changes. In the solution of the equation, the amplitude parameters are used for y, and the pulse width parameters are used for x. The intercept b on the y or AMP axis can then be found, which is shown as $A_I$ in FIGURE 8. Each time new pulse parameters are needed for an output pulse, one parameter is used in the equation to calculate the other.

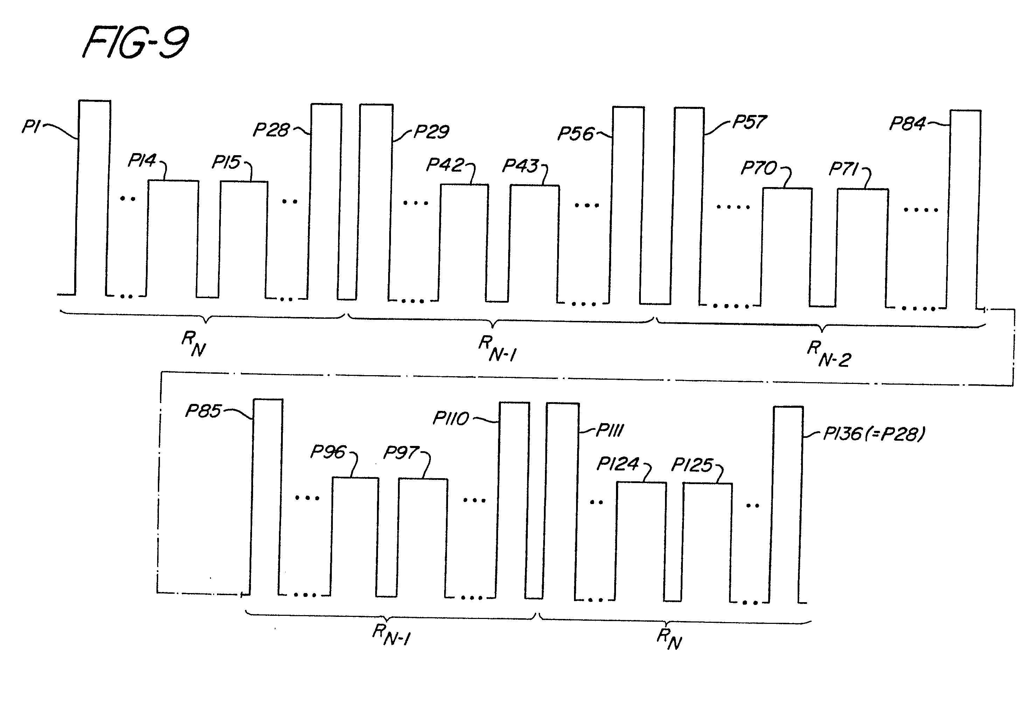

During the modulation mode, 28 pulses are produced at the pulse rate entered by the patient. The first fourteen pulses exhibit decreasing amplitudes and increasing pulse widths. The next fourteen pulses reverse this process, and exhibit increasing amplitudes and decreasing pulse widths so that the 28th pulse exhibits the same amplitude and pulse width as the starting pulse. The first, fourteenth, fifteenth and 28th pulses are shown in FIGURE 9 as pulses P1, P14, P15, and P28, respectively, with intervening pulses occurring during the dotted portions of the sequence. This first pulse sequence is at the starting pulse rate of $R_N$.

Another sequence of 28 pulses is then produced at the next lower pulse rate of the device, $R_{N-1}$. These pulses exhibit the same pulse width and amplitude variations as the first sequence. A third sequence of 28 pulses is produced at the next lower pulse rate, $R_{N-2}$, again with the amplitude and pulse width variations.

Eighty-four pulses, P1-P84, have now been produced. During the next two rate sequences the rate decrease is reversed. The sequence of pulses P85-P110 is produced at rate $R_{N-1}$, and the final sequence at rate $R_N$. The last illustrated pulse P136 is at an equivalent point in the rate cycling as pulse P28, from which point the sequencing continues as treatment progresses.

A constructed embodiment of the present invention is capable of producing pulses at either of ten different rates: 2, 10, 20, 30, 40, 50, 60, 70, 80 or 100 Hz. If the starting rate is 20 or above, the rate cycling will be as described in FIGURE 9. If a rate of 10 Hz is chosen, however, there is only one possible lower rate. Hence, under these conditions, the rate modulation will only vary between the lower two rates of 10 Hz and 2 Hz. Similarly, if a starting rate of 2 Hz, the lowest rate, is chosen, the system will be unable to drop to any lower rate, and pulses of changing width and amplitude will be produced continuously at the 2 Hz rate.

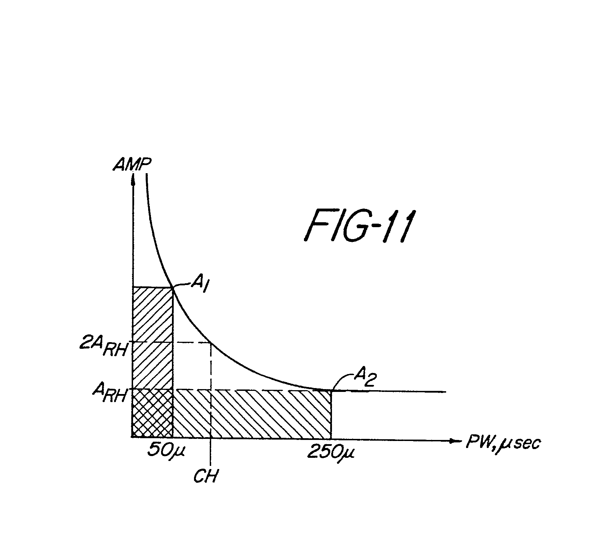

The strength-duration mode, as mentioned above, is a clinician's tool, used to try ones of a family of pulse sequences. A typical strength-duration characteristic curve is shown in FIGURE 10. This curve is closely described by the equation

$$I = \frac{I_{RH}}{1-e^{-t/k}}$$

28

where I is pulse intensity, t is the pulse width, $I_{RH}$ is the intensity when the pulse width is large (the rheobase), and k is a constant. The pulse width when the intensity is twice the rheobase ($2I_{RH}$), shown as $t_{CH}$ is called the chronaxie.

The stimulation device of the present invention uses input parameters from the operator to calculate a strength-duration curve. The operator sets the mode switch 24 to the strength-duration mode, and turns on the stimulation device. A different strength-duration curve may be calculated for either output in the same manner as follows.

The operator first uses the DISP button to display the AMP indicator for the selected output. The pulse width is internally set to 50 µsec by the background program, and the rate is internally set to 80 Hz. The operator then pushes the SET button and, using the INC and DEC buttons, selects a desired amplitude $A_1$ for a 50 µsec pulse.

The operator pushes the SET button a second time, and the preceding parameters are stored by the background program. The program then internally sets the pulse width to 250 µsec and displays a zero amplitude. The operator then uses the INC and DEC buttons to select an amplitude value $A_2$ for a 250 µsec pulse. The operator pushes the SET button a third time. The 250 µsec pulse parameters are stored and used together with the 50 µsec pulse parameters in the calculation of the strength-duration curve.

The strength-duration curve is calculated from the amplitude values $A_1$ and $A_2$ and their respective pulse widths which were previously entered by the operator.

STM-28

These parameters are shown for the two pulses in
FIGURE 11. These parameters represent two solutions for
the equation

$$A = \frac{A_{RH}}{1-e^{-PW/k}}$$

This equation is solved by successive approximation, using
$A_1$ and $A_2$ for A in the equation, and 50 μsec and 250 μsec
for PW. The rheobase, $A_{RH}$ and the constant k will
then be found. The constant k is a function of the
chronaxie, being equal to 1.44 times the chronaxie. Once
the rheobase and the constant k are known, the parameters
at any point along the curve can be found. Thus, when the
microprocessor has solved the equation, it displays the PW
indicator on the display. The operator will enter a pulse
width value, and the device will respond by producing an
80 Hz pulse train with an amplitude corresponding to the
entered pulse width value on the strength-duration curve.
The operator can continue to change the pulse width value
until a comfortable pulse width/amplitude sequence is
found.

Since the operator is free to use any amplitude values
when setting up the strength-duration curve, it is
possible for values to be entered which cannot be fitted
to a logarithmic curve described by the equation. For
instance, this condition will arise if the amplitude $A_1$ of
the 50 μsec pulse is far greater than the amplitude $A_2$
selected for the 250 μsec pulse. If this happens, the
background program will progressively decrease the $A_1$
amplitude until a curve fit with $A_2$ is found. This will
occur, as a practical matter, whenever the ratio of $A_1$ to
$A_2$ is greater than five. Lesser ratios will result in a
curve fit using the operator-entered values. This type of

curve-fitting scheme will protect the patient from high value pulses resulting from the selection of a large $A_1$ value for the 50 µsec pulse.

A logarithmic curve will also not be found if the amplitude entered for the 250 µsec pulse is greater than that entered for the 50 µsec pulse. In that circumstance, the stimulation device will produce a pulse sequence with the amplitude chosen for the 250 µsec pulse for any pulse width entered by the operator. This choice is based upon the premise that a pulse amplitude which the patient can experience without pain at 250 µsec can also be safely withstood at lesser pulse widths.

Other aspects of the operation of the microprocessor controlled transcutaneous nerve stimulation device shown in Figure 1 are described and claimed in currently filed European patent application No.           , entitled TRANSCUTANEOUS NERVE STIMULATION DEVICE USING A COMMON CONTROLLER FOR PULSE PRODUCTION AND PARAMETER DISPLAY (Reference STM 26, claiming priority from USSN 617 442).

STM-28

CLAIMS

1.    A method of producing a treatment pulse sequence in a transcutaneous nerve stimulation device comprising the steps of:

a)    producing a first sequence of pulses at a first rate, during which the pulse amplitude and pulse width are modulated; and

b)    producing a second sequence of pulses at a second rate, during which the pulse amplitude and pulse width are modulated.

2.    The method of Claim 1, wherein step b) is followed by the step of:

c)    producing a third sequence of pulses at a third rate, during which the pulse amplitude and pulse width are modulated.

3.    The method of Claim 2, wherein step c) is followed by the steps of:

d)    repeating step b); and

e)    repeating step a); and

f)    cycling continuously through steps b), c), d) and e).

4.    The method of claim 2 or claim 3, wherein said second rate is less than said first rate and said third rate is less than said second rate.

-24-

5.    The method of any one of claims 1 to 4, wherein pulse amplitude and width are modulated in the same manner in each sequence.

6.    The method of Claim 5, wherein during each sequence the pulse amplitude is sequentially decreased from a starting value, then increased back to said starting value, while the pulse width is sequentially increased from a starting value then decreased back to said starting value.

7.    The method of Claim 6, wherein the minimum pulse amplitude is equal to the starting amplitude value less 40% of the starting amplitude value, and the maximum pulse width is equal to the starting pulse width plus 60% of the starting pulse width.

8.    The method of any one of claims 1 to 7 wherein said stimulation device includes a controller responsive to the input of pulse parameter values, and wherein step a) is preceded by the step of

g)    inputting into said controller pulse parameters of amplitude, width, and rate from which the modulation of pulse amplitude and width and the changing of rate from one sequence to the other are determined.

9 . A method of producing a treatment pulse sequence in a transcutaneous nerve stimulation device which includes a processor responsive to the input of pulse parameter values comprising the steps of:

a) inputting into said processor the parameters of two treatment pulses;

b) calculating from said parameters a strength-duration curve which contains the loci of a family of pulse parameters including those of said two treatment pulses; and

c) producing a treatment pulse sequence in which said treatment pulses exhibit parameters located on said strength-duration curve.

10. The method of Claim 9 , wherein step b) comprises:

b) calculating a strength-duration curve described by the equation

$$ I = \frac{I_{RH}}{1-e^{-t/k}} $$

using values for amplitude I and pulse width t for the two pulses input in step a) to find a rheobase constant $I_{RH}$ and a chronaxie constant k.

11. The method of Claim 10 , wherein step c) comprises:

c) producing a treatment pulse sequence in which said treatment pulses exhibit parameters located on said strength-duration curve by inputting one of a pulse amplitude value or pulse width value located on said curve

28

and solving the equation of step b) for the remaining parameter.

12. The method of any one of claims 9 to 11 wherein steps a) and c) comprise the steps of

a)  inputting into said processor the amplitude parameters for two treatment pulses of predetermined pulse width parameters;  and

c)  selecting a pulse width parameter located on said strength-duration curve to calculate the corresponding amplitude parameter on the curve so as to produce a treatment pulse sequence exhibiting the selected and calculated pulse parameters.

FIG-1

# FIG-2

MICRO-PROCESSOR μPD80C49G

BUS 1: P10, P11, P12, P13, P14, P15, P16, P17

BUS 2: P20, P21, P22, P23, P24, P25, P26, P27

BUS 3: DB0, DB1, DB2, DB3, DB4, DB5, DB6, DB7
WR 32, EN1 33, EN2 34, CLK 38, SHR 36, AMPI 37, REFG 31, MSW 35

X1, X2, RESET, T0, T1, INT.

DISP, INC, DEC, SET

FROM LOW BATTERY DETECT

POWER ON INIT.

FROM SENTINEL

10, 101, 103, 105

FIG-3

0171881

# FIG-4

0171881

FIG-5

## FIG-6A

```
 LOC
 007
   │
   ▼
MOV R7, A
   │
   ▼
SEL. RBI
   │
   ▼
TIMER=240
   │
   ▼
DECREMENT
LCD CTR
   │
   ▼
LCD CTR=0? ──Y──> LCD CTR=8 ──> OUTPUT LCD DATA ──> INVERT FI
   │ N
   ▼
TO FIG.6B
```

DECREMENT POWER ON CTR

POWER ON CTR=99?  ──N──>  POWER ON CTR=0?  ──N──>

Y (POWER ON CTR=99?) ──> TURN OFF LED

Y (POWER ON CTR=0?) ──> POWER ON CTR=100 ──> TURN ON LED

FROM FIG. 6A

**FIG-6B**

TO FIG. 6C

*Flowchart nodes:*

R2 SENTINEL FAILURE? — Y → TURN ON LED; N → DECREMENT RATE CTR

DECREMENT RATE CTR → RATE CTR = 0?

RATE CTR = 0? — Y → RATE CTR = RATE; N → RATE CTR = 2?

RATE CTR = 2? — Y → STORE LCD DATA; N → RESTORE LCD DATA

STORE LCD DATA → AMP1 > 4ma?

AMP1 > 4ma? — Y → OUTPUT SENTINEL 1; N →

OUTPUT SENTINEL 1 → AMP2 > 4ma?

AMP2 > 4ma? — Y → OUTPUT SENTINEL 2; N →

OUTPUT SENTINEL 2 →

IF INTERRUPT, SET SENTINEL R2

MODULATE MODE? — Y → SET FLAG TO CALCULATE NEXT PULSE; N →

BURST MODE? — Y → IS BURST OVER?; N → (NORMAL OR S-D MODE)

IS BURST OVER? — N → CLEAR BURST FLAG; Y →

STORE LCD DATA

RESTORE LCD DATA ← OUTPUT 0's TO D/A CONV.

OUTPUT PULSE 1 / OUTPUT PULSE 2

7/12

0171881

FROM FIG. 6B

DECREMENT ½ SEC CTR

½ SEC CTR = 0 ? —Y→ ½ SEC CTR = 200 → SET TREATMENT TIME FLAG; CLEAR ½ SEC FLAG; CLEAR SAMPLE MODE SW FLAG

N

BURST MODE SELECTED ? —Y→ DECREMENT BURST CTR → BURST CTR = 0 ? —Y→ BURST CTR = 200 → SET BURST FLAG

N (BURST CTR = 0 ?)

N (BURST MODE SELECTED ?)

SEL RBØ

MOV A, R7

RETR

FIG-6C

## FIG-7

FIG-8

FIG-10

0171881

FIG-9

0171881

FIG-11

European Patent Office

**PARTIAL EUROPEAN SEARCH REPORT**
which under Rule 45 of the European Patent Convention
shall be considered, for the purposes of subsequent
proceedings, as the European search report

**0171881**
Application number

## DOCUMENTS CONSIDERED TO BE RELEVANT

EP 85303924.6

| Category | Citation of document with indication, where appropriate, of relevant passages | Relevant to claim | CLASSIFICATION OF THE APPLICATION (Int. Cl.4) |
|---|---|---|---|
| X | <u>FR - A1 - 2 336 145</u> (GNUDDE) | 1,5 | A 61 N 1/36 |
| A | * Page 1, lines 12-17; page 2, lines 5-9; fig. 2 * | 4 | |
| | -- | | |
| X | <u>GB - A - 1 546 089</u> (LIM BOON CHEN) | 1 | |
| | * Page 1, lines 43-45; page 4, claim 1 * | | |
| | -- | | |
| X | <u>AT - B - 352 866</u> (LOHER) | 1,2,4, 5,8 | |
| | * Page 2, lines 12-17, 39-49; fig. * | | |
| | -- | | |
| A | <u>US - A - 4 442 839</u> (MAURER) | 1,2,4, 5,8 | |
| | * Abstract; column 1, lines 29-34, 47-55; column 1 line 65 - column 2, line 8; fig. 3 * | | TECHNICAL FIELDS SEARCHED (Int. Cl.4) |
| | -- | | A 61 N |

### INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely: 1-8

Claims searched incompletely: 9-12

Claims not searched: ▬

Reason for the limitation of the search:

according to Art. 52(2)(a)

| Place of search | Date of completion of the search | Examiner |
|---|---|---|
| VIENNA | 22-11-1985 | NEGWER |

EPO Form 1505.1 03.82

European Patent Office

**PARTIAL EUROPEAN SEARCH REPORT**

EP 85303924.6

## DOCUMENTS CONSIDERED TO BE RELEVANT

| Category | Citation of document with indication, where appropriate, of relevant passages | Relevant to claim |
|---|---|---|
| X | US - A - 3 626 926 (MIKHAIL ILICH KUZIN) <br> * Abstract * <br> -- | 1 |
| A | US - A - 3 902 502 (LISS) <br> * Abstract; fig. * <br> -- | 1,5 |
| A | DE - A - 2 416 401 (SONO-THERAPY) <br> * Page 26, claims 1,2 * <br> -- | 1,2,4,5 |
| A | EP - A1 - 0 099 662 (WILMOT) <br> * Abstract; page 2, line 26 - page 3, line 1; page 6, claims 1-8 * <br> -- | 1,2,4,5 |
| A | EP - A2 - 0 010 364 (STIMTECH) <br> * Abstract; page 3, lines 24-37; page 11, claims 1-5 * <br> ---- | 1,2,4,5 |

**CLASSIFICATION OF THE APPLICATION (Int. Cl.4)**

**TECHNICAL FIELDS SEARCHED (Int. Cl.4)**